# EUROPEAN PATENT APPLICATION

(11) **EP 4 378 351 A1**
(43) Date of publication of application: **05.06.2024**
(21) Application number: 23152377.0
(22) Date of filing: 19.01.2023
(51) Int. Cl.: A46B 15/00, A61C 17/22

(54) **AN ORAL CARE DEVICE**

(30) Priority: 02.12.2022 US 202263429625 P
(71) Applicant: Koninklijke Philips N.V., 5656 AG Eindhoven (NL)
(72) Inventor: VAN HULTEN, Maaike Cornelia Johanna Wilhelmina, Eindhoven (NL); HORSTMAN, Pieter, Eindhoven (NL); GOTTENBOS, Bart, Eindhoven (NL)
(74) Representative: Philips Intellectual Property & Standards

(57) **Abstract**

An oral care device has a light source for delivering UV or visible treatment light. A location sensor tracks a position and orientation of the oral care device so that a region of the oral cavity location to which the treatment light is being delivered can be determined. The amount of treatment light delivered by the light source to each region is monitored during an oral care routine and the light source can thus be controlled to ensure a light dose to each region remains below a threshold.

## Description

### FIELD OF THE INVENTION

This invention relates to oral care devices, and in particular oral care devices which incorporate a light treatment function.

### BACKGROUND OF THE INVENTION

Oral health diseases are still one of the most prevalent issues in the western world, despite a multitude of high-quality oral cleaning products and increased and detailed knowledge on the necessity for oral hygiene.

Caries as well as gingivitis, or in more severe cases periodontitis, are often a result of insufficient oral hygiene alone or in combination with genetic predisposition. Next to manual treatments including increased oral hygiene regimens, drugs like antibiotics or tissue stimulating proteins are used for treating e.g. periodontitis.

A relatively new method to obtain a healthy oral cavity, additional to brushing, is the application of light. Ultraviolet light has a wavelength from 100nm to 400nm. There are three main forms of UV light: UV-A (long-wave 315 to 400nm), UV-B (medium-wave 280 to 315nm) and UV-C (shortwave 100nm to 280nm). UV-C is harmful for the skin and is used to sterilize surfaces and can kill viruses and bacteria. This property can also be used in the oral cavity by applying light e.g. via a toothbrush or mouthpiece to kill bacteria.

However, by using UV-C in the mouth, the tissues in the oral cavity can also be damaged depending on the dose applied, which is of course an undesirable side effect. Light sources in the visible light spectrum, near the UV spectrum (i.e. at the short wavelength violet end of the visible spectrum), have been tested using widely available LED sources of violet light (418nm) and blue light (453nm) both of which also show bactericidal or bacteriostatic effect.

Incorporation of light in oral devices has already been proposed. It is necessary to determine the optimal intensity and treatment time for bacteria and also to test the effect of the light source on cell cultures. In particular, the applied dose of light should not exceed a threshold, above which cells will be damaged. There is therefore a need for an effective safety feature to prevent a light overdose. This would be desirable for both visible light treatment and UV light treatment.

### SUMMARY OF THE INVENTION

The invention is defined by the claims.

According to examples in accordance with an aspect of the invention, there is provided an oral care device comprising:
a UV or visible light source for delivering treatment light to the oral cavity of a user, the oral cavity comprising a set of regions;
a location sensor for providing location information comprising a position and orientation of the oral care device;
a controller for controlling the light source and receiving the location information,
wherein the controller is configured to:
determine, from the location information, the region of the oral cavity location to which the treatment light is being delivered;
monitor the amount of treatment light delivered by the light source to each region during an oral care routine;
control the light source to ensure a light dose to each region remains below a threshold.

This oral care device monitors the region to which treatment light is delivered during an oral care routine. By tracking the time and intensity of light delivered to each region of the oral cavity, it can be ensured that no region experiences a light dose greater than a threshold. In this way, cell damage, resulting from exceeding a dose limit, can be avoided. Thus, short term harm to the user can be prevented.

Each region for example corresponds in size to a treatment area when the light source is static relative to the oral cavity. However, each region may instead comprise an area including a relatively small amount of movement of the device and hence the light source.

The threshold is for example 30 J/cm² or less. A dose greater than 30 J/cm² , for example 60 J/cm², is found to cause cell damage, particularly for violet and blue visible light treatment.

The controller is for example configured to switch off the light source when the location information indicates that the treatment light is being delivered outside the oral cavity.

Thus, the light source can be switched off automatically once the device is outside the oral cavity. This prevents that light can shine in the user's eye, which is also an unsafe situation for the user (particularly for visible light).

The location sensor for example comprises an inertia monitoring unit. It may combine an accelerometer (e.g. 3 axis) and a gyroscope (e.g. 3 axis). This tracks absolute position and orientation, once a starting position and orientation is known.

The controller is for example configured to control the light source by switching the light source on and off. This provides a simple way to provide dose control.

The controller may additionally or alternatively be configured to control the light source by controlling the light intensity when the light source is on. This enables more accurate dose control over time. For example, the light intensity may be controlled in dependence on an allocated treatment time.

The controller may be configured to control the light source using historical information relating to previous oral care routines performed by the user. This historical information enables a more personalized approach, which takes account of regions the user tends to miss or only cover for a short time during their oral care routine (so that a higher intensity light for a shorter time may be appropriate).

For example, the controller may be configured to identify from the historical information one or more regions which are regularly missed during the oral care routine of the user. These may be considered to be problem spots.

The device may further comprise a feedback device for providing feedback to a user to indicate that a region has already been treated with a maximum light dose. This enables the user to focus on other areas during their oral care routine.

The device may further comprise an oral health sensor for detecting an oral health condition at the individual regions, wherein the controller is configured to control the light source further in dependence on the oral health condition.

The oral health sensor for example comprises one or more of:
a plaque sensor;
a blood sensor.

The device is for example a powered toothbrush or a cleaning mouthpiece or a light treatment device.

The invention also provides a computer program comprising computer program code means which is adapted, when said program is run on a controller of an oral care device, to implement a method comprising:
controlling a UV or visible light source of the oral care device to deliver treatment light to the oral cavity of a user, the oral cavity comprising a set of regions;
determining, from location information comprising a position and orientation of the oral care device received from a location sensor of the oral care device, the region of the oral cavity location to which the treatment light is being delivered;
monitoring the amount of treatment light delivered by the light source to each region during an oral care routine; and
controlling the light source to ensure a light dose to each region remains below a threshold.

The invention also provides a method of controlling an oral care device, which device comprises a UV or visible light source for delivering treatment light to the oral cavity of a user, the oral cavity comprising a set of regions and a location sensor for providing location information comprising a position and orientation of the oral care device, wherein the method comprises:
controlling the light source to deliver treatment light to the oral cavity of a user, the oral cavity comprising a set of regions;
determine, from location information comprising a position and orientation of the oral care device received from the location sensor, the region of the oral cavity location to which the treatment light is being delivered;
monitor the amount of treatment light delivered by the light source to each region during an oral care routine; and
control the light source to ensure a light dose to each region remains below a threshold.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows experimental results of testing cytotoxicity for different doses of violet light;
Fig. 2 shows an example of an oral care device in the form of a powered toothbrush;
Fig. 3 shows a first dose control method;
Fig. 4 shows a second dose control method;
Fig. 5 shows a third dose control method; and
Fig. 6 shows a processor architecture that may be used by the controller of the oral care device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an oral care device having a light source for delivering treatment light. A location sensor tracks a position and orientation of the oral care device so that a region of the oral cavity to which the treatment light is being delivered can be determined. The amount of light delivered by the light source to each region is monitored during an oral care routine and the light source can thus be controlled to ensure a light dose to each region remains below a threshold.

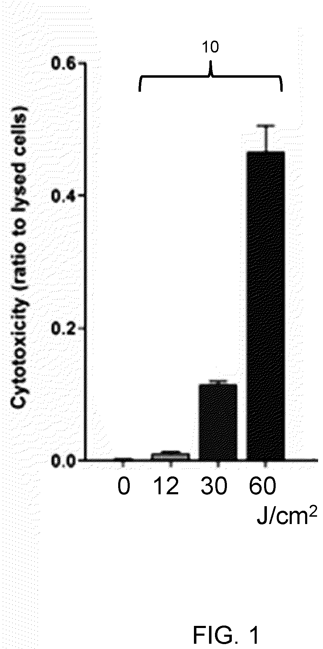

Fig. 1 shows experimental results of testing cytotoxicity for different doses (0 J/cm², 12 J/cm², 30 J/cm² and 60 J/cm²). The light source had a (violet) wavelength of 418nm at 100 mW/cm² intensity. The set of plots 10 are for GFB-16 cells (immortalized gingival fibroblast).

A dose of 30 J/cm² already results in an increase in cytotoxicity level but a dose of 60 J/cm² results in a significant increase in cytotoxicity level.

It is estimated from these experiments that there is a safety limit for the dosage of light to be used in the oral cavity which is preferably set at 30 Joules/cm² or even set at a lower level, per treatment. The actual threshold will depend on the wavelength of light used.

The invention is based on the recognition that the safety limit should be applied per region of the oral tissue rather than setting a maximum dose for the overall treatment. In particular, if there is a fixed overall treatment dose, a user will not apply the treatment uniformly to all areas of the oral cavity so that some regions may be exposed to an excessive dose.

Instead, the invention makes use of location sensing so that per-region dosage may be monitored. For this purpose, a location sensor such as an inertia monitoring unit, IMU, can be used to determine if a specific region, i.e. a particular treatment spot, already has achieved the maximum amount of treatment light.

The treatment spot (i.e. the area covered by the illumination) preferably has a size corresponding to a fraction of the size of a tooth. The resolution is limited by both the detector resolution (the IMU and the image recognition system) and the size of the illuminated surface area that can be controlled.

The regions may be pre-defined, so that the oral cavity is segmented into a default set of regions at the outset. Alternatively, a mapping may be built up over time which defines the regions. Personalization is possible over time as the system learns the geometry of the particular user.

The area covered by the irradiation depends on the optical design, such as the use of a single LED or an array of LEDs.

The location sensing is used to monitor the position and orientation of the oral care device relative to the user's oral cavity. By itself, an IMU will give an absolute position and orientation of the device (given a known starting position). An AI algorithm can be used to derive the desired relative position information. Indeed, such location processing is already used in existing electric toothbrushes, such as known Sonicare (trade mark) toothbrushes. The AI algorithm for processing location sensing information is trained to a ground truth. The AI is thereby self-calibrating, based on known and possible user behavior on which it is trained. When using a camera, relative orientation can be derived by comparing images with images of known locations.

The light source can then be deactivated on a per-region basis so that the safe dosage limit is not exceeded at any region of the oral cavity. This is currently a risk for consumer-orientated treatment devices. The intensity of the light may also be adapted at any particular region in order to boost the efficacy momentarily, while still remaining in a safe dosage regime. This feature can be activated when it is detected that e.g. there is still dental plaque left and (additional) treatment is necessary.

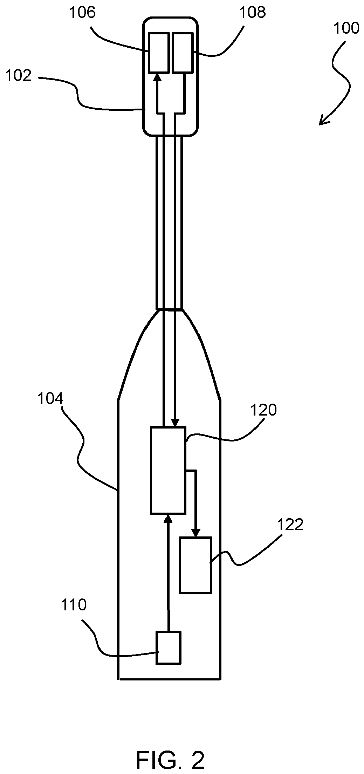

Fig. 2 shows an example of an oral care device in the form of a powered toothbrush 100. The invention may however be applied to a cleaning mouthpiece or a stand-alone light treatment unit. For the case of a cleaning mouthpiece, the light illumination is for example performed using an array of individually controlled light elements (e.g., LEDs). A one-size fits all mouthpiece needs to be moved by the user to find the best fit. This means not all teeth will receive the same dose of light, and the location sensing can be used to monitor the local light dose. Also, some mouthpieces do not treat both sides or both arches at the same time, so that again location-specific control is of interest.

The toothbrush comprises a cleaning head 102 which is driven by a motor (not shown) in the handle 104. The cleaning head includes a treatment light source 106 and an optional oral health sensor 108. The light source is for example a 418nm light source or a 453nm light source for providing treatment light. The wavelength is thus near the UV end of the visible light spectrum in these examples. However, the invention may equally be applied to UV light, e.g. UV-C light.

A location sensor 110 is provided in the handle for sensing a position and orientation of the device, which in turn indicates the orientation and position of the light source, and hence the region of the user's oral cavity that is illuminated by the light source.

The location sensor may be placed in or on the device main body, or it may be designed to be snapped on to the device as an external sensor,

The light source 106 is controlled by a controller 120. The controller 120 also receives the location information from the location sensor 110. This information comprises a position and orientation of the oral care device. The location sensor is for example a 6-axis inertia monitoring unit, IMU, i.e., a 3-axis accelerometer and a 3-axis gyroscope. The IMU measures and reports the acceleration and orientation of the device.

The IMU and the light source can be situated in different parts of the toothbrush. For example, the light source may be a click-on LED that communicates with the IMU in the toothbrush handle.

A first control approach is to monitor and store location information and also store the associated dose delivered to the corresponding region of the oral cavity. The cumulative dose during an oral care routine may thus be determined. A determination is then made whether to switch on or off the light source depending on the region of the oral cavity faced by the light source.

A second control approach is to adapt the light intensity of the light source to enable a higher or lower light intensity, for example depending on an allocated treatment time still reserved for a particular treatment region. Thus, the dose control can also take account of the intended treatment time at different regions. The user for example is instructed to follow a particular care routine with certain treatment times allocated to different oral cavity regions.

A third control approach is a refinement to the second approach in which an oral health measure, such as plaque detection, is used to control the amount of light to be applied to a specific region.

The device of the invention ensures that a maximum light dosage is not exceeded at any specific region. The device can also provide information to the user about their oral care routine, because the location is tracked. For example, the user may be educated about regions they tend to miss. Feedback may also be used to motivate the user to improve his/her brushing technique because the visual change in light intensity will indicate where brushing has already been completed. Plaque can be treated effectively while maintaining a safe dosage regime.

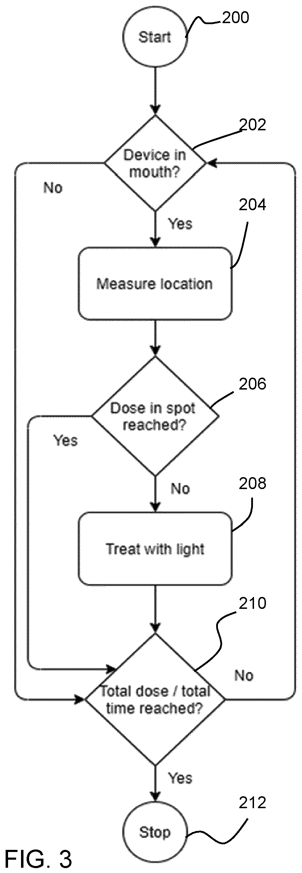

Fig. 3 shows how the device is operated in the first control approach mentioned above.

The toothbrush is started in step 200.

In step 202, it is detected if the toothbrush is in the mouth. This can be achieved via e.g., a measurement that distinguishes between an in-mouth location and an outside mouth location e.g., conductivity, temperature, light, force measurements, also combined with the fact that the device has been switched on. In addition, sensed movement of the IMU can be used, e.g., the motion corresponding to picking up the device and placing it in the mouth could be detected.

Once the toothbrush is in the mouth, the light source is activated and location sensing takes place. The time during which light is delivered to each region of the oral cavity, and the intensity (if there is an adjustable intensity) is monitored, so that a total per-region dose is established. If the intensity is adjustable, the controller sets the intensity, so a feedback measure is not needed. For a fixed intensity approach, the maximum dose of course corresponds to a maximum per-region treatment time.

For each region of the oral cavity, until the maximum dose is reached as monitored in step 206, light treatment continues to take place in step 208. When the threshold dose is reached for any particular region, the treatment step is bypassed.

The treatment continues until a total treatment dose is reached or a total treatment time is reached, as determined in step 210, The method then ends in step 212.

If at any time it is detected (in step 202) that the device is no longer in the mouth, the light treatment is again bypassed (until the device is once again in the mouth).

Feedback to the user can be given, for example using an acoustic noise (buzz), a light signal (e.g. flashing), a software feedback signal (e.g. in-app signals) or a vibration, as soon as a region has already been treated to the maximum extent. The user then knows that he/she should move the device to another region. Another specific signal can be given to the user when the whole oral cavity is treated.

Fig. 1 shows a feedback device 122 for providing this feedback to a user, such as to indicate that a region has already been treated with a maximum light dose.

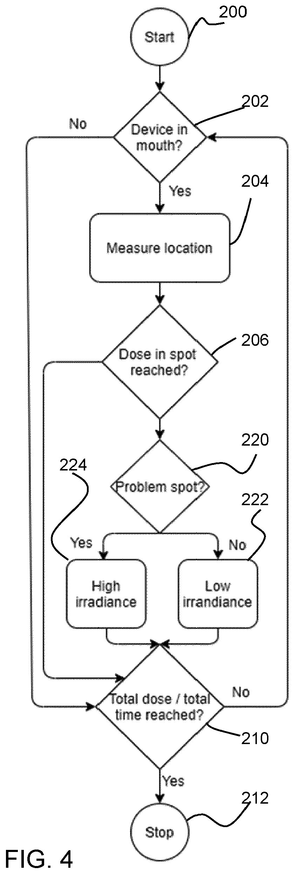

Fig. 4 shows how the device is operated in the second control approach mentioned above. The same steps are given the same reference numbers as in Fig. 3.

In this method, the light treatment in step 208 is enhanced by detecting problem spots, which are regions the user tends to miss during their oral care routine. In particular, the light intensity can be adjusted, depending on the location and orientation of the oral care device, to provide an improved light treatment for these problem areas.

For this purpose, information from previous oral care sessions, stored in a memory, is used to provide an input to determine if a specific region is missed on a regular basis, and could therefore be indicated to be a problem area. A dental professional (e.g. dentist or oral hygienist) can appoint a specific area to be a problem area and indicate this in an app so that the oral device knows that this area needs special attention.

It is determined if the current location corresponds to a problem area in step 220. If the location does not correspond to a problem area, a low irradiance is used in step 222 whereas if the location does correspond to a problem area, a high irradiance is used in step 224. The requirement that the maximum dose is not exceeded is maintained.

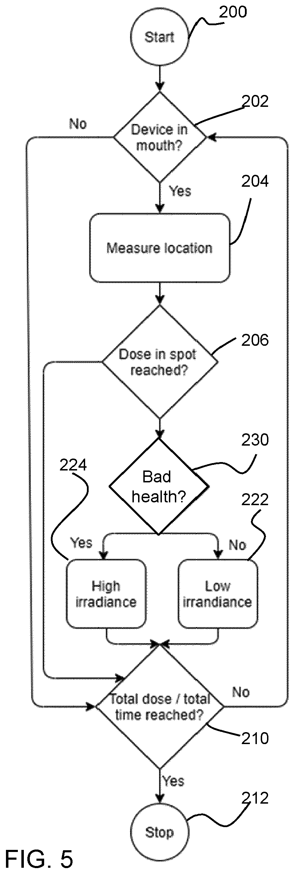

Fig. 5 shows how the device is operated in the third control approach mentioned above. The same steps are given the same reference numbers as in Figs. 3 and 4.

Instead of determining that the current location corresponds to a problem region in step 220, a health measure is obtained in step 230. For this purpose, a plaque sensor may be used detect the presence of plaque and thereby indicate if a specific region is missed (because there is plaque present) and/or still needs attention. Alternatively, or additionally. a blood sensor detects the presence of blood which could indicate the presence of gingivitis or periodontitis. This is also an indication that more care is needed for these regions.

If one or more such conditions are detected (denoted as "bad health") in step 230, high irradiance is used in step 224 whereas for already-healthy tissue low irradiance is used in step 222.

A combination of information obtained by the IMU and the oral health sensor (plaque detection sensor and/or blood detection sensor) is thereby used to determine if the light treatment can continue or must stop because the maximum dose is reached on that specific region.

The invention applies generally to oral care devices (e.g. mouthpiece, toothbrush, or stand-alone light treatment device) with a light treatment feature. The treatment light is intended to kill bacteria in the dental plaque and so reduce the amount of oral plaque.

As discussed above, the system makes use of controller to perform the data processing. The processor can be implemented in numerous ways, with software and/or hardware, to perform the various functions required. The controller typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

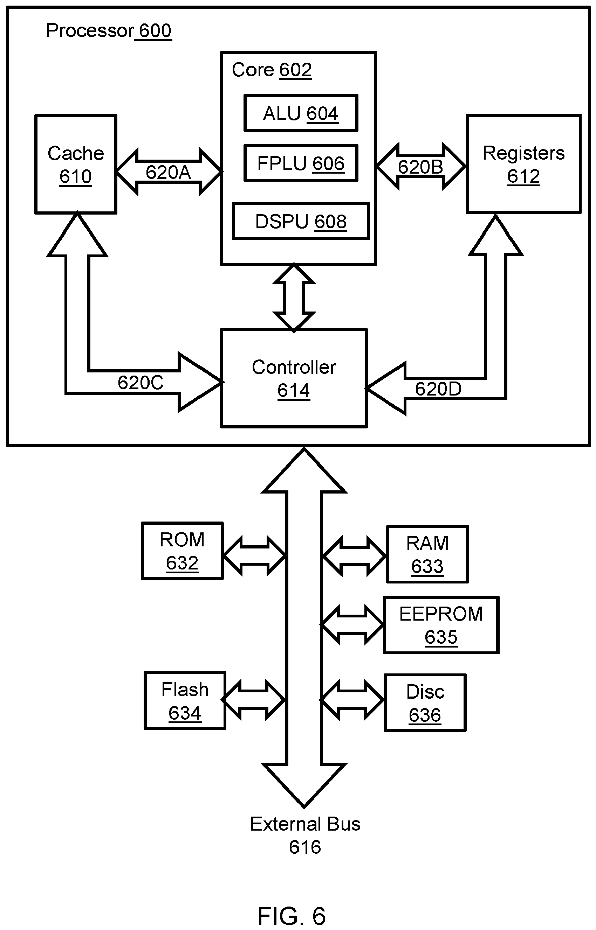

Fig. 6 is a block diagram illustrating an example processor 600 according to embodiments of the disclosure. Processor 600 may be used to implement one or more processors described herein, for example, controller 120 shown in Fig. 2. Processor 600 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 600 may include one or more cores 602. The core 602 may include one or more arithmetic logic units (ALU) 604. In some embodiments, the core 602 may include a floating-point logic unit (FPLU) 606 and/or a digital signal processing unit (DSPU) 608 in addition to or instead of the ALU 604.

The processor 600 may include one or more registers 612 communicatively coupled to the core 602. The registers 612 may be implemented using dedicated logic gate circuits (e.g., flip-flops) and/or any memory technology. In some embodiments the registers 612 may be implemented using static memory. The register may provide data, instructions and addresses to the core 602.

In some embodiments, processor 600 may include one or more levels of cache memory 610 communicatively coupled to the core 602. The cache memory 610 may provide computer-readable instructions to the core 602 for execution. The cache memory 610 may provide data for processing by the core 602. In some embodiments, the computer-readable instructions may have been provided to the cache memory 610 by a local memory, for example, local memory attached to the external bus 616. The cache memory 610 may be implemented with any suitable cache memory type, for example, metal-oxide semiconductor (MOS) memory such as static random-access memory (SRAM), dynamic random-access memory (DRAM), and/or any other suitable memory technology.

The processor 600 may include a controller 614, which may control input to the processor 600 from other processors and/or components included in a system and/or outputs from the processor 600 to other processors and/or components included in the system. Controller 614 may control the data paths in the ALU 604, FPLU 606 and/or DSPU 608. Controller 614 may be implemented as one or more state machines, data paths and/or dedicated control logic. The gates of controller 614 may be implemented as standalone gates, FPGA, ASIC or any other suitable technology.

The registers 612 and the cache 610 may communicate with controller 614 and core 602 via internal connections 620A, 620B, 620C and 620D. Internal connections may implemented as a bus, multiplexor, crossbar switch, and/or any other suitable connection technology.

Inputs and outputs for the processor 600 may be provided via a bus 616, which may include one or more conductive lines. The bus 616 may be communicatively coupled to one or more components of processor 600, for example the controller 614, cache 610, and/or register 612. The bus 616 may be coupled to one or more components of the system.

The bus 616 may be coupled to one or more external memories. The external memories may include Read Only Memory (ROM) 632. ROM 632 may be a masked ROM, Electronically Programmable Read Only Memory (EPROM) or any other suitable technology. The external memory may include Random Access Memory (RAM) 633. RAM 633 may be a static RAM, battery backed up static RAM, Dynamic RAM (DRAM) or any other suitable technology. The external memory may include Electrically Erasable Programmable Read Only Memory (EEPROM) 635. The external memory may include Flash memory 634. The External memory may include a magnetic storage device such as disc 636.

A computer program carrier may comprise a relatively permanent storage device (e.g. hard drives, solid state drives etc.) and/or may comprise a temporary carrier (e.g. a bitstream etc.).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An oral care device comprising:
a UV or visible light source (106) for delivering treatment light to the oral cavity of a user, the oral cavity comprising a set of regions;
a location sensor (110) for providing location information comprising a position and orientation of the oral care device;
a controller (120) for controlling the light source and receiving the location information, wherein the controller is configured to:
determine, from the location information, the region of the oral cavity location to which the treatment light is being delivered;
monitor the amount of light delivered by the light source to each region during an oral care routine;
control the light source to ensure a light dose to each region remains below a threshold.

2. The device of claim 1, wherein the threshold is 30 J/cm² or less.

3. The device of claim 1 or 2, wherein the controller (120) is configured to switch off the light source when the location information indicates that the treatment light is being delivered outside the oral cavity.

4. The device of any one of claims 1 to 3, wherein the location sensor (110) comprises an inertia monitoring unit.

5. The device of any one of claims 1 to 4, wherein the controller (120) is configured to control the light source by switching the light source on and off.

6. The device of any one of claims 1 to 5, wherein the controller (120) is configured to control the light source by controlling the light intensity when the light source is on.

7. The device of claim 6, wherein the controller (120) is configured to control the light intensity in dependence on an allocated treatment time.

8. The device of any one of claims 1 to 7, wherein the controller (120) is configured to control the light source using historical information relating to previous oral care routines performed by the user.

9. The device of claim 8, wherein the controller (120) is configured to identify from the historical information one or more regions which are regularly missed during the oral care routine of the user.

10. The device of any one of claims 1 to 9, further comprising a feedback device (122) for providing feedback to a user to indicate that a region has already been treated with a maximum light dose.

11. The device of any one of claims 1 to 10, further comprising an oral health sensor (108) for detecting an oral health condition at the individual regions, wherein the controller is configured to control the light source further in dependence on the oral health condition.

12. The device of claim 11, wherein the oral health sensor (108) comprises one or more of:
a plaque sensor;
a blood sensor.

13. The device of any one of claims 1 to 12, comprising a powered toothbrush or a cleaning mouthpiece or a light treatment device.

14. A computer program comprising computer program code means which is adapted, when said program is run on a controller of an oral care device, to implement a method comprising:
controlling a UV or visible light source of the oral care device to deliver treatment light to the oral cavity of a user, the oral cavity comprising a set of regions;
determining, from location information comprising a position and orientation of the oral care device received from a location sensor of the oral care device, the region of the oral cavity location to which the treatment light is being delivered;
monitoring the amount of treatment light delivered by the light source to each region during an oral care routine; and
controlling the light source to ensure a light dose to each region remains below a threshold.

15. A method of controlling an oral care device, which device comprises a UV or visible light source for delivering treatment light to the oral cavity of a user, the oral cavity comprising a set of regions and a location sensor for providing location information comprising a position and orientation of the oral care device, wherein the method comprises:
controlling the light source to deliver treatment light to the oral cavity of a user, the oral cavity comprising a set of regions;
determine, from location information comprising a position and orientation of the oral care device received from the location sensor, the region of the oral cavity location to which the treatment light is being delivered;
monitor the amount of treatment light delivered by the light source to each region during an oral care routine; and
control the light source to ensure a light dose to each region remains below a threshold.
